Europäisches Patentamt

European Patent Office

Office européen des brevets

(11) Publication number: **0 329 297**
**A2**

# EUROPEAN PATENT APPLICATION

(21) Application number: **89300867.2**

(51) Int. Cl.⁴: **G01N 21/64 , A61B 5/00**

(22) Date of filing: **30.01.89**

(30) Priority: **16.02.88 US 155964**

(43) Date of publication of application:
**23.08.89 Bulletin 89/34**

(84) Designated Contracting States:
**AT BE CH DE ES FR GB IT LI NL**

(71) Applicant: **MEDEX, INC.**
**3637 Lacon Road**
**Hilliard Ohio 43026(US)**

(72) Inventor: **Davis, Robert C.**
**1723 Grace Lane**
**Columbus Ohio(US)**
Inventor: **Barnes, Russell H.**
**1478 Berkshire Road**
**Columbus Ohio(US)**
Inventor: **Nicholson, Warren B.**
**1906 Queensbridge Drive**
**Columbus Ohio(US)**

(74) Representative: **Allen, Oliver John Richard et al**
**Lloyd Wise, Tregear & Co. Norman House**
**105-109 Strand**
**London, WC2R 0AE(GB)**

(54) **Method and apparatus for measuring partial pressure of oxygen in a fluid.**

(57) A sensor 10 that includes a fluorescing dye is contacted by a fluid containing the unknown quantity of oxygen. The dye is excited by a light pulse 18. The rate of decay of the light 24 produced by the excited dye is measured and is converted to the pressure of the oxygen in the fluid.

EP 0 329 297 A2

## Method and Apparatus for Measuring Partial Pressure of Oxygen in a Fluid Abstract

This invention relates to a method and apparatus for measuring partial pressure of oxygen ($PO_2$), and particularly relates to the in vivos measurement of the partial pressure of oxygen in the blood of humans.

U.S. Patent No. 4,476,870, discloses a method and apparatus for measuring oxygen in human blood. The patent describes a probe or sensor which is inserted in the oxygen-containing fluid (blood). The probe contains a fluorescent dye that is sensitive to oxygen quenching.

The dye is excited by a blue light and fluoresces. The fluorescence, a green light, is measured and the ratio of the intensity of the excitation light to the green light is a measure of the quenching effect of the oxygen in the blood. The problem with the Peterson method and other methods similar to it is that the calculation of blood pressure rests upon a determination of the absolute intensity of excitation and the intensity of the fluorescence. There are factors in the system that can cause variation of the intensity of fluorescence that are not easily controlled. The aging of the dye is one of them. The amount of dye is another. The quality of the matrix holding the dye is still another. Therefore, when measuring the level of intensity of fluorescence of the dye, the operator cannot be sure that the conditions have not varied from standard conditions under which the apparatus was calibrated.

It is an object of the present invention to provide a method and apparatus for measuring the partial pressure of oxygen in a fluid such as human blood and to eliminate the effects of variables that may cause variations in the intensity of the fluorescence of the excited dye.

This object with the invention is attained in accordance by measuring the rate of decay of the fluorescence. If a known fluorescing dye is excited by a pulse of light of known wavelength to cause the dye to fluoresce, the intensity of fluorescence will always decay at the same rate regardless of the level of intensity attained. Thus, if the rate of decay of the level of intensity is measured, it will be unaffected by any extraneous factors that would tend to vary the absolute level of intensity of the fluorescing dye.

Preferably, a dye such as fluoranthene in a matrix of silicone rubber is inserted into the $O_2$ containing fluid. The dye is excited by a short pulse (3 nanoseconds). The dye will fluoresce and the emitted light will be received at its maximum intensity in about 30 nanoseconds from the time that the laser source emits its pulse. The intensity of the light at that point in time is measured and stored. After a certain period, 10 nanoseconds for example, the intensity of emitted light is again measured and stored. The difference between the two values is a measure of the quenching effort of the $O_2$ and hence the partial pressure of the $O_2$.

As a preliminary to the application of the invention, a series of samples having known, gradually varying amounts of oxygen are monitored in apparatus as disclosed above, thereby producing a series of decay rates. This series of decay rates can be stored in a computer as a "look-up table" against which the decay rate for an unknown sample is compared. Or, alternatively, an equation based on a curve of the known decay rates versus oxygen pressures can be prepared and stored in the computer as the basis for determining the oxygen pressure of the sample.

An apparatus in accordance with the present invention is characterised by a sensor containing a fluorescable substance, means for subjecting the substance to a pulse of light of a preselected wavelength, so exciting the substance to fluorescence, means for measuring the intensity of fluorescence, as it decays, at two discrete intervals in time and means for converting the rate of decay of intensity to the partial pressure of oxygen.

The light source of the apparatus may be a pulse laser, which generates a pulse preferably 3-5 nanoseconds in duration. The measuring means of the apparatus preferably comprises means for converting the intensity of the fluorescence to a voltage to which a pair of track or hold amplifiers are connected to receive a decaying voltage proportional to the intensity of fluorescence. A pair of time delay units are triggered by the emitted pulse of light and cause the voltage corresponding to the intensity to be held by the track or hold amplifiers at two discrete intervals in time, determined by the time delay units.

The present invention will now be described by way of example with reference to the accompanying drawings in which:

Fig. 1 is a block diagram of the optical and electrical apparatus in accordance with the present invention;

Fig. 2 is a graph of several curves of fluorescence intensity versus time for several $O_2$ bearing samples; and

Fig. 3 is a curve of oxygen pressures versus decay rates.

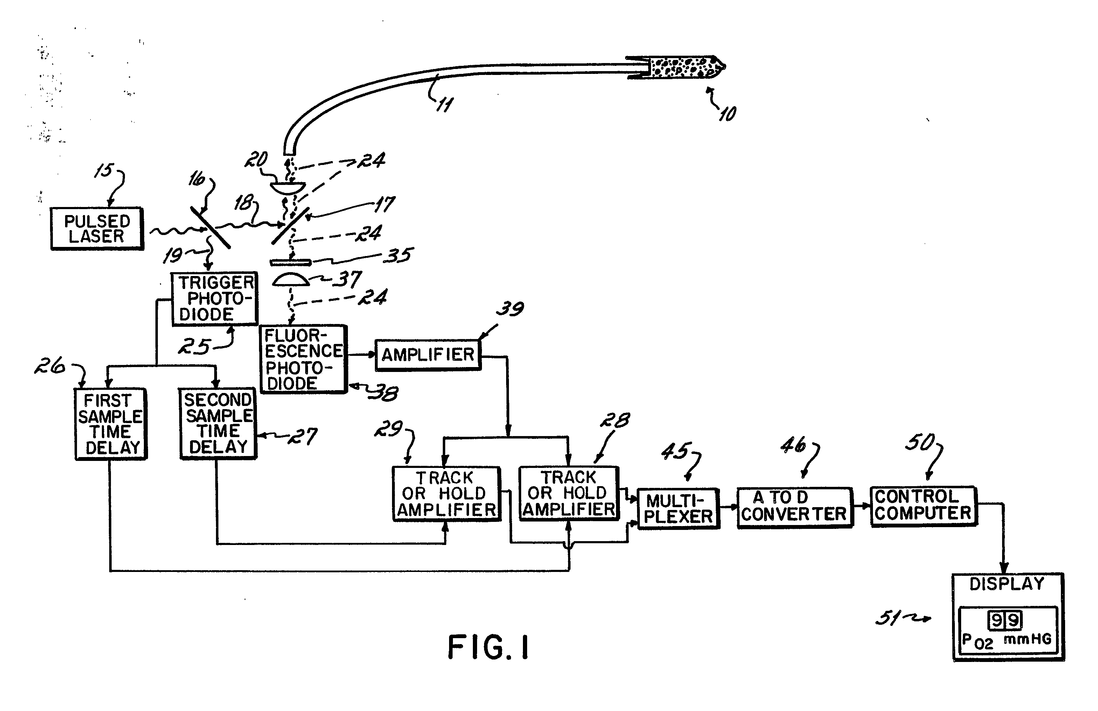

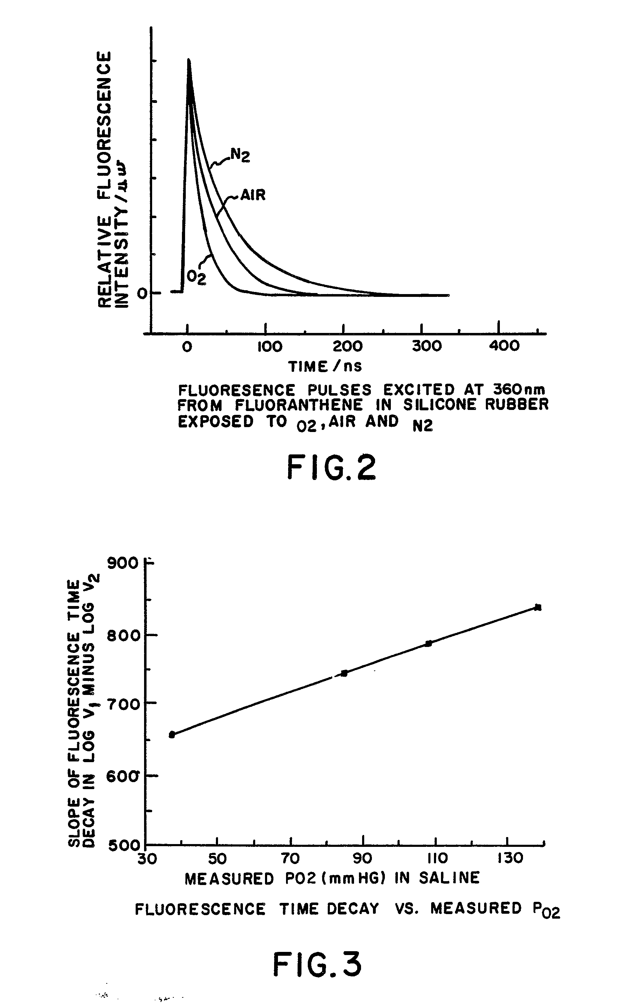

Referring to Fig. 1, a sensor 10 is connected to the end of an optical fiber 11. The sensor 10 may be any material that fluoresces when excited and whose fluorescence is quenched by oxygen, for example, perylene dibutyrate or fluoranthene carried in an silicone rubber matrix. A source of pulsed laser 15 is positioned adjacent a first beam splitter 16 and a second beam splitter 17. The first beam splitter 16 passes approximately 90% of its energy, indicated as being 18, and reflects approximately 10% of its energy, indicated as being 19, at right angles. The beam splitter 17 is specially coated to reflect approximately 100% of the beam of the laser pulse 18 through a lens 20 into the optical fiber 11. The pulse excites the dye causing it to fluoresce or luminesce, the wavelength of luminescence being in the blue-green portion of the spectrum. That fluorescence will immediately decay. The slope of its decay curve will vary with the percentage of $O_2$ in the fluid. Three representative curves for 0%, 21% and 100% $O_2$ are shown in Fig. 2. The beam splitter 17 is adapted to pass substantially 100% of that emitted wavelength.

Adjacent the beam splitter 16 is a trigger photodiode 25 that is connected to first and second time delay units. The time delay units 26 and 27 are connected respectively to track or hold amplifiers 28 and 29. Adjacent the beam splitter 17 is an interference filter 35 that passes the blue-green emitted beam 24. A lens 37 focuses the beam on a fluorescence diode 38 that converts the intensity of the beam 24 to a voltage proportional to the intensity. The output of the photodiode 38 is connected through an amplifier 39 to the track or hold amplifiers 28 and 29. When the track or hold amplifiers are triggered by their respective time delay units 26 and 27, they will hold the level of voltage received from amplifier 39. The output of each track and hold amplifier is at a voltage level proportional to the intensity of the beam 24 at the times that the time delay units 26 and 27 triggered the track or hold amplifiers 28 and 29. The respective outputs of the amplifiers 28 and 29 are connected to a multiplexer 45 which feeds a signal to an analog-to-digital converter 46, the output of which is fed to a computer 50. The computer has a display 51 associated with it which preferably is in mmHg units so as to display, in substantially real time, the actual partial pressure of oxygen-bearing fluid in which the sensor 10 is introduced.

In a representative operation of the apparatus of Fig. 1, the sensor 10 is introduced into the bloodstream of the patient. The laser source 15 will send a pulse of approximately 3 nanoseconds duration through beam splitter 16 to beam splitter 17. Beam splitter 17 directs the pulse through the lens 20 which focuses it onto the optical fiber 11. The pulse excites the dye in the sensor 10, causing it to fluoresce. the fluorescence, at a wavelength of 360 nanometers, passes through the optical fiber, lens 20, beam splitter 17, filter 35 and lens 37 to the fluorescence photodiode 38 where it is converted to a voltage. The intensity of the fluorescence decays in a curve such as is shown in Fig. 2. The voltage output of the photodiode decays proportionately. That decaying voltage is amplified by amplifier 39, the output of which is connected to the two track and hold amplifiers.

Approximately 30 nanoseconds are required for the light to travel up the optical fiber to excite the dye in the sensor and down the optical fiber to the fluorescence diode 38. Assuming that the fluorescence at diode 38 will therefore be a peak intensity at approximately 30 nanoseconds, the first time delay unit 26 is adapted to trigger its track or hold amplifier 28 at 30 nanoseconds. An interval of about 10 nanoseconds will show measurable decay of this intensity of the fluorescence and that level will be on a linear portion of the curve of Fig. 2. Therefore, the time delay unit 27 is set to trigger the track or hold amplifiers 29 at 40 nanoseconds after the emission of the laser pulse to provide an interval of 10 nanoseconds for decay. The voltages held on amplifiers 28 and 29 are fed through multiplexer 45 and analog-to-digital converter 46 to computer 50. The computer calculates the log of the first voltage and subtracts from it the log of the second voltage. This information is a measure of the slope of the intensity curve for a specific pressure of oxygen. The computer computers that slope in an equation representing a curve of all of the known slopes of relevant oxygen pressures (Fig. 3) and outputs on the display 51 the partial oxygen pressure of the sample contacted by the sensor.

As indicated, Fig. 2 show representative curves of the emission intensities of fluoranthene when contacted by zero oxygen ($N_2$), 21% ocygen (air) and 100% oxygen, respectively.

Fig. 3 is a curve of the oxygen partial pressure versus the slopes of all curves between 0 and 100%. To prepare the curve of Fig. 3, the logarithmic values of the voltages at 30 nanoseconds and 40 nanoseconds are subtracted to provide a slope value for each oxygen pressure. Those slope values are plotted in the curve of Fig. 3. The curve of Fig. 3 is converted to an equation which the computer simply solves to provide an output on the display of oxygen pressure for any input of voltage levels corresponding to first and second intensity levels samples by the system.

In this description, a pulse length of 3 nanoseconds, and triggering at 30 and 40 nanoseconds has been disclosed. It should be understood that these values are only representative of an operative set of conditions and can be varied within the scope of the invention.

## Claims

1. A method to determine the partial pressure of oxygen $O_2$ in fluid having an unknown concentration of oxygen comprising radiating a fluorescable substance with a pulse of light to cause the substance to fluoresce characterised in that the intensity of the fluorescence is measured at two discrete time intervals, the rate of decay of the intensity is determined and compared with known standard rates of decay of intensity for the same fluorescable substance in environments containing known concentrations of oxygen.

2. A method as claimed in claim 1 comprising the calibration of the measurements by measuring the standard rates of decay of intensity of the fluorescable substance when subjected to a pulse of light causing it to fluoresce characterised in that the fluorescable substance is in the presence of a fluid containing a known concentration of oxygen and repeating the calibration process with fluids of different known concentrations, so forming a family of decay rates for that fluorescable substance.

3. Apparatus for determining the partial pressure of $O_2$ in a fluid having an unknown concentration of oxygen characterised by a sensor containing a fluorescable substance, means for subjecting the substance to a pulse of light of a preselected wavelength, so exciting the substance to fluorescence, means for measuring the intensity of fluorescence, as it decays, at two discrete intervals in time and means for converting the rate of decay of intensity to the partial pressure of oxygen.

4. Apparatus as claimed in claim 3 in which the fluorescable substance is a dye.

5. Apparatus as claimed in claim 3 or 4 in which the subjecting means comprises, a source of pulsed laser, generating a pulse of about 3-5 nanoseconds in duration.

6. Apparatus as claimed in any of claims 3-5 in which the measuring means includes, means for converting the intensity of the fluorescence to a voltage, a pair of time delay units each having a respective output connected to a track or hold amplifier, the time delay units having a short and long time delay, respectively, and trigger means connecting the pulse to the time delay units to initiate operation of the time delay units to cause, in turn, the track or hold amplifiers to hold the respective values of voltage after the short and long time delays, respectively.

FIG.1

EP 0 329 297 A2

FLUORESENCE PULSES EXCITED AT 360nm
FROM FLUORANTHENE IN SILICONE RUBBER
EXPOSED TO O₂, AIR AND N2

FIG.2

FLUORESCENCE TIME DECAY VS. MEASURED $P_{O2}$

FIG.3